# EUROPEAN PATENT APPLICATION

(11) **EP 4 403 178 A1**
(43) Date of publication of application: **24.07.2024**
(21) Application number: 22870049.8
(22) Date of filing: 16.09.2022
(51) Int. Cl.: A61K 31/7048, A23L 33/105, A23L 33/125, A61P 19/02, A61P 43/00, C12N 5/077

(54) **PROMOTER FOR DIFFERENTIATION INTO CARTILAGE CELL, CARTILAGE CELL PROPAGATION PROMOTER, AND CARTILAGE MATRIX PRODUCTION PROMOTER**

(30) Priority: 17.09.2021 JP 2021152615
(71) Applicant: Hokuriku University, Kanazawa-shi, Ishikawa 920-1180 (JP); Cokey Systems Co., Ltd., Matsusaka-shi, Mie 515-0041 (JP)
(72) Inventor: MIURA, Masakazu, Kanazawa-shi, Ishikawa 920-1181 (JP); TAKAHASHI, Tatsuo, Kanazawa-shi, Ishikawa 920-1181 (JP); SUZUKI, Hirokazu, Kanazawa-shi, Ishikawa 920-1181 (JP); YOSHIKAWA, Nobuji, Matsusaka-shi, Mie 515-0041 (JP)
(74) Representative: Weickmann & Weickmann PartmbB
(86) International application number: PCT/JP2022/034704
(87) International publication number: WO 2023/042902

(57) **Abstract**

The present invention relates to promoters for differentiation into chondrocytes, chondrocyte proliferation promoter, and cartilaginous matrix production promoter, each containing a glycoside of liquiritigenin.

According to the present invention, promoters for differentiation into chondrocytes, promoters for proliferation of chondrocytes, and promoters for cartilaginous matrix production, that are superior in the stability and safety, have a superior promoting action on differentiation into chondrocytes, a superior promoting action on proliferation of chondrocytes, and a superior promoting action on cartilaginous matrix production, and effective for regeneration of cartilage and further, prophylaxis or improvement of symptoms and diseases caused by the damage and inflammation of cartilage, or symptoms and diseases showing the loss of cartilage such as osteoarthritis and the like.

## Description

### [Technical Field]

The present invention relates to promoters for differentiation into chondrocytes, promoters for proliferation of chondrocytes, and promoters for cartilaginous matrix production that promote differentiation of chondrogenic progenitor cells into chondrocytes, and can further promote proliferation of chondrocytes and production of cartilaginous matrix.

### [Background Art]

Cartilage is a connective tissue consisting of chondrocytes and a matrix surrounding them. It is known that aging, congenital or acquired joint abnormalities, trauma such as ligament rupture, excessive mechanical stress caused by weight increase and the like, and the like causes a decrease in the number of chondrocytes and a decline in their function, which in turn degenerate cartilage tissue and develop diseases such as osteoarthritis.

For such diseases, symptomatic therapy such as administration of anti-inflammatory drugs; conservative therapy such as topical rest, orthotic therapy, intra-articular injection of hyaluronic acid and the like; and surgical therapy such as arthroscopic bone marrow stimulation, osteochondral autograft transplantation, autologous chondrocyte implantation, high tibial osteotomy and the like have been applied.

However, symptomatic therapy and conservative therapy are not fundamental treatment methods. Among surgical treatments, arthroscopic bone marrow stimulation and osteochondral autograft transplantation target small-scale cartilage defects, and are not suitable for application to large-scale cartilage defects such as severe knee osteoarthritis. Furthermore, in autologous chondrocyte implantation, since the patient's own cells need to be collected from the joint, the problem of invasiveness has been pointed out. Furthermore, in this transplantation procedure, the collected cells need to be drastically amplified, but the problem of dedifferentiation has also been pointed out since differentiated chondrocytes may lose their original functions when they are amplified.

Therefore, the search for components that promote chondrocyte differentiation or chondrocyte proliferation, or the production of cartilaginous matrix or components that promote cartilage regeneration is underway, and C16N, which is a protein called "neurochondrin", C16N-1 and C16N-2, which are isoforms of C16N, or proteins similar to these proteins (Patent Literature 1), peptides and proteins that are osteoclast differentiation factor (RANKL) acting substances (Patent Literature 2), harmine or a salt thereof (Patent Literature 3), plant-derived components such as Mube (Stauntonia hexaphylla)leaf extract (Patent Literature 4), 20-hydroxyecdysone-containing plant extract (Patent Literature 5), and the like have been reported.

However, there are concerns about problems in sensitizing property and formulation stability with respect to proteins and peptides, and plant-derived components are not sufficiently satisfactory in terms of stability, safety, effect, and the like.

Therefore, a differentiation promoter and the like that are superior in stability and safety, have a superior promoting action on differentiation into chondrocytes or a superior promoting action on proliferation of chondrocytes or a superior promoting action on cartilaginous matrix production, and useful for regeneration of cartilage and further, treatment of osteoarthritis and the like have been demanded.

### [Citation List]

### [Patent Literature]

[Patent Literature 1]
   WO 2000/001405
[Patent Literature 2]
   WO 2010/038610
[Patent Literature 3]
   JP-A- 2012-171947
[Patent Literature 4]
   Japanese Translation of PCT Application Publication No. 2016-520059
[Patent Literature 5]
   JP-A- 2016-210767

### [Summary of Invention]

### [Technical Problem]

Thus, the present invention aims to provide promoters for differentiation into chondrocytes, chondrocyte proliferation promoters, and cartilaginous matrix production promoters, that are superior in stability and safety, have a superior promoting action on differentiation into chondrocytes and a superior promoting action on proliferation of chondrocytes, and further, a superior promoting action on cartilaginous matrix production, and useful for regeneration of cartilage and further, prophylaxis or improvement of symptoms and diseases caused by the damage and inflammation of cartilage such as osteoarthritis and the like, or symptoms and diseases showing the loss of cartilage.

### [Solution to Problem]

The present inventors have found and already disclosed that liquiritigenin contained in licorice extract promotes differentiation of chondrogenic progenitor cells into chondrocytes, further promotes proliferation of chondrocytes, and promotes production of cartilaginous matrix (Japanese patent application No. 2020-079740).

They have now found that liquiritigenin glycosides have a superior differentiation promoting action from chondrogenic progenitor cells into chondrocytes and the like, and conducted further studies and completed the present invention.

Accordingly, the present invention relates to the following.
[1] A promoter for differentiation into chondrocytes, comprising a glycoside of liquiritigenin.
[2] The promoter described in [1], wherein the glycoside of liquiritigenin is liquiritin.
[3] A promoter for chondrocyte proliferation, comprising a glycoside of liquiritigenin.
[4] The promoter described in [3], wherein the glycoside of liquiritigenin is liquiritin.
[5] A cartilaginous matrix production promoter comprising a glycoside of liquiritigenin.
[6] The promoter described in [5], wherein the glycoside of liquiritigenin is liquiritin.
[7] A cartilage regenerant comprising a glycoside of liquiritigenin.
[8] The regenerant described in [7], wherein the glycoside of liquiritigenin is liquiritin.
[9] A pharmaceutical product for the prophylaxis or improvement of a symptom or disease caused by damage or inflammation of cartilage, or a symptom or disease showing the loss of cartilage, comprising the differentiation promoter of [1] or [2] .
[10] The pharmaceutical product described in [9], wherein the symptom or disease caused by damage or inflammation of cartilage, or the symptom or disease showing the loss of cartilage is osteoarthritis.
[11] A pharmaceutical product for the prophylaxis or improvement of a symptom or disease caused by damage or inflammation of cartilage, or a symptom or disease showing the loss of cartilage, comprising the proliferation promoter described in [3] or [4].
[12] The pharmaceutical product described in [11], wherein the symptom or disease caused by damage or inflammation of cartilage, or the symptom or disease showing the loss of cartilage is osteoarthritis.
[13] A pharmaceutical product for the prophylaxis or improvement of a symptom or disease caused by damage or inflammation of cartilage, or a symptom or disease showing the loss of cartilage, comprising the production promoter of [5] or [6] .
[14] The pharmaceutical product described in [13], wherein the symptom or disease caused by damage or inflammation of cartilage, or the symptom or disease showing the loss of cartilage is osteoarthritis.
[15] A food for the prophylaxis or improvement of a symptom or disease caused by damage or inflammation of cartilage, or a symptom or disease showing the loss of cartilage, comprising the differentiation promoter of [1] or [2].
[16] The food described in [15], wherein the symptom or disease caused by damage or inflammation of cartilage, or the symptom or disease showing the loss of cartilage is osteoarthritis.
[17] A food for the prophylaxis or improvement of a symptom or disease caused by damage or inflammation of cartilage, or a symptom or disease showing the loss of cartilage, comprising the proliferation promoter described in [3] or [4].
[18] The food described in [17], wherein the symptom or disease caused by damage or inflammation of cartilage, or the symptom or disease showing the loss of cartilage is osteoarthritis.
[19] A food for the prophylaxis or improvement of a symptom or disease caused by damage or inflammation of cartilage, or a symptom or disease showing the loss of cartilage, comprising the production promoter of [5] or [6].
[20] The food described in [19], wherein the symptom or disease caused by damage or inflammation of cartilage, or the symptom or disease showing the loss of cartilage is osteoarthritis.
[21] A method for promoting differentiation into chondrocyte or proliferation of chondrocyte, comprising ingestion by or administration to a target animal in need of promotion of differentiation into chondrocyte or proliferation of chondrocyte, of a glycoside of liquiritigenin in an amount effective for promoting differentiation into chondrocyte or proliferation of chondrocyte in the target animal.
[22] The method described in [21], wherein the glycoside of liquiritigenin is liquiritin.
[23] A method for promoting cartilaginous matrix production, comprising ingestion by or administration to a target animal in need of promotion of cartilaginous matrix production, of a glycoside of liquiritigenin in an amount effective for promoting cartilaginous matrix production in the target animal.
[24] The method described in [23], wherein the glycoside of liquiritigenin is liquiritin.
[25] A method for regenerating cartilage, comprising ingestion by or administration to a target animal in need of cartilage regeneration, of a glycoside of liquiritigenin in an amount effective for regenerating cartilage in the target animal.
[26] The method described in [25], wherein the glycoside of liquiritigenin is liquiritin.
[27] A method for preventing or improving cartilage reduction or loss, comprising ingestion by or administration to a target animal in need of preventing or improving cartilage reduction or loss, of a glycoside of liquiritigenin in an amount effective for preventing or improving cartilage reduction or loss in the target animal.
[28] The method described in [27], wherein the glycoside of liquiritigenin is liquiritin.
[29] A method for preventing or improving a symptom or disease caused by damage or inflammation of cartilage, or a symptom or disease showing the loss of cartilage, comprising ingestion by or administration to a target animal in need of preventing or improving a symptom or disease caused by damage or inflammation of cartilage, or a symptom or disease showing the loss of cartilage, of a glycoside of liquiritigenin in an amount effective for preventing or improving said symptom or disease in the target animal.
[30] The method described in [29], wherein the glycoside of liquiritigenin is liquiritin.
[31] The method described in [29] or [30], wherein the symptom or disease caused by damage or inflammation of cartilage, or the symptom or disease showing the loss of cartilage is osteoarthritis.

### [Advantageous Effects of Invention]

According to the present invention, promoters for differentiation into chondrocytes, promoters for proliferation of chondrocytes, and promoters for cartilaginous matrix production, which have high stability and high safety and are superior in a promoting action on differentiation into chondrocytes, a promoting action on proliferation of chondrocytes, and a promoting action on cartilaginous matrix production, can be provided.

The promoters for differentiation into chondrocytes, promoters for proliferation of chondrocytes, and promoters for cartilaginous matrix production of the present invention are effective for regeneration of cartilage, and useful for the prophylaxis or improvement of symptoms and diseases caused by damage or inflammation of cartilage, or symptoms and diseases showing the loss of cartilage, such as osteoarthritis and the like.

### [Brief Description of Drawings]

[Fig. 1]

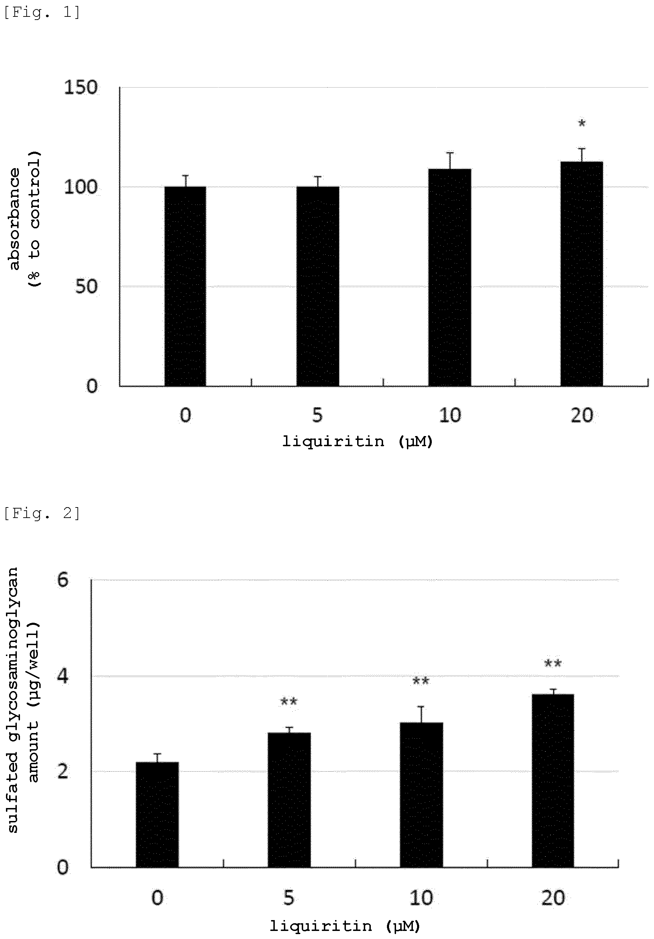

   Fig. 1 is a diagram showing that in Experimental Example 1, cells on day 24 of culture were stained with Alcian blue, and the amount of Alcian blue dye solubilized from the cells was measured by absorbance at 620 nm and shown as a relative value to the control. In the Figure, "*" means significant against control at p<0.05.
[Fig. 2]
   Fig. 2 is a diagram showing the content of sulfated glycosaminoglycan in the digestive fluid of cells on day 24 of culture in Experimental Example 1. In the Figure, "**" means significant against control at p<0.01.
[Fig. 3]

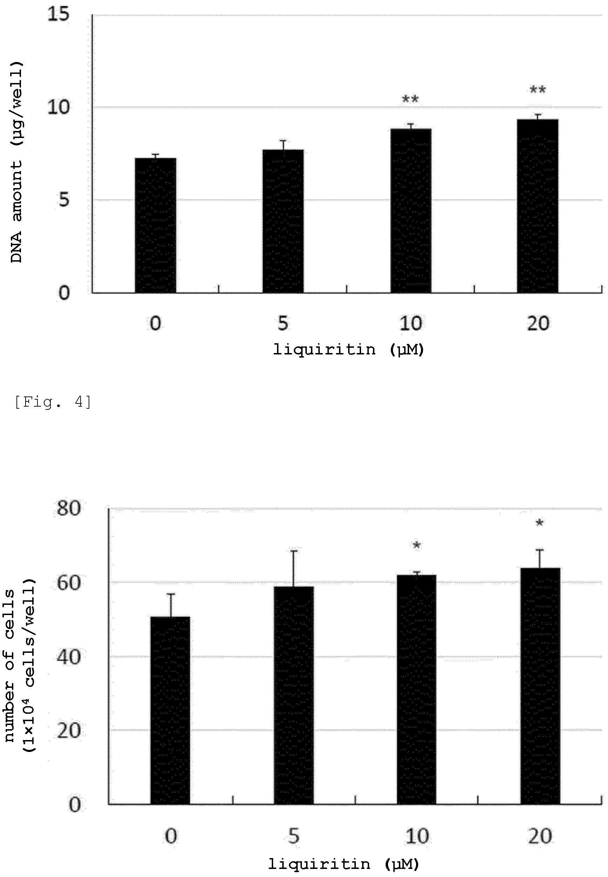

   Fig. 3 is a diagram showing the content of DNA in the digestive fluid of cells on day 24 of culture in Experimental Example 1. In the Figure, "**" means significant against control at p<0.01.
[Fig. 4]
   Fig. 4 is a diagram showing the number of cells on day 24 of culture in Experimental Example 1. In the Figure, "*" means significant against control at p<0.05.

### [Description of Embodiments]

The present invention provides promoters for differentiation into chondrocytes (hereinafter sometimes referred to as "the differentiation promoter of the present invention" in the present specification), proliferation promoters for chondrocytes (hereinafter sometimes referred to as "the proliferation promoter of the present invention" in the present specification), and production promoters for cartilaginous matrix (hereinafter sometimes referred to as "the production promoter of the present invention" in the present specification).

The differentiation promoter, proliferation promoter, and production promoter of the present invention contain a glycoside of liquiritigenin as the active ingredient.

The glycoside of liquiritigenin contained as the active ingredient in the differentiation promoter, proliferation promoter, and production promoter of the present invention is flavanone glycoside contained in licorice and, for example, liquiritin ((2S)-2,3-dihydro-7-hydroxy-2-[4-(β-D-glucopyranosyl oxy)phenyl]-4H-1-benzopyran-4-one) represented by the following chemical formula (1), liquiritin apioside((2S)-4'-(2-O-D-apio-β-D-furanosyl-β-D-glucopyranosyl oxy)-7-hydroxyflavanone) represented by the following chemical formula (2), glucoliquiritin apioside((2S)-4'-(2-O-D-apio-β-D-furanosyl-β-D-glucopyranosyl oxy)-7-(β-D-glucopyranosyl oxy)flavanone) represented by the following chemical formula (3), neoliquiritin ((S)-2,3-dihydro-7-(β-D-glucopyranosyl oxy)-2-(4-hydroxyphenyl)-4H-1-benzopyran-4-one) represented by the following chemical formula (4), and the like can be mentioned.

In the present invention, one kind of glycoside of liquiritigenin may be selected and used alone, or two or more kinds thereof can also be selected and used in combination.

From the aspect of the promoting effect on the differentiation into chondrocytes and the like, liquiritin is preferably used in the present invention.

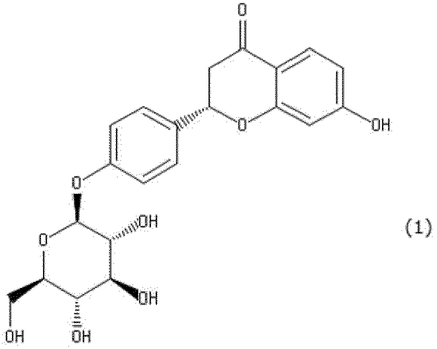

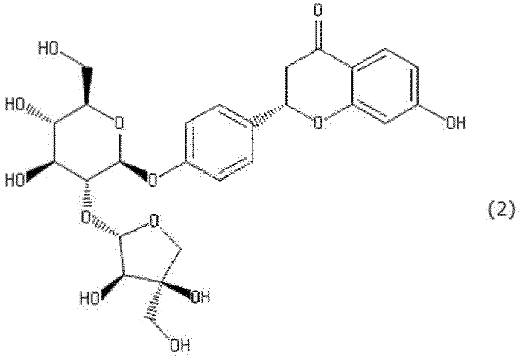

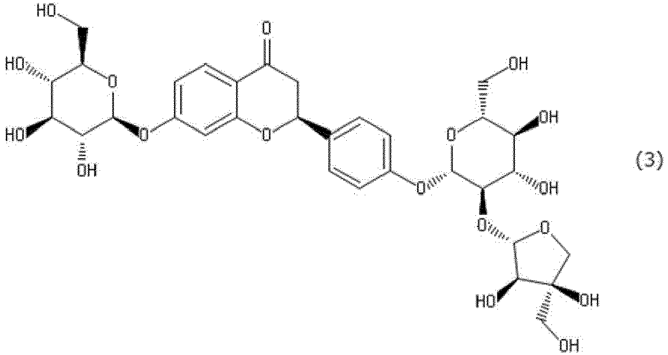

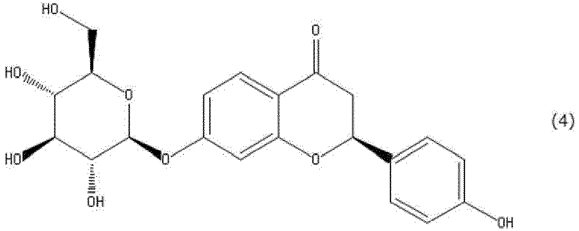

In the present invention, purified products provided by various companies can be used as the glycoside of liquiritigenin, and a fraction of licorice extract containing a high content of glycoside of liquiritigenin, such as liquiritin, can also be used.

When using a fraction of licorice extract, it is desirable to use a fraction that does not contain glycyrrhizin, which is the main medicinal component of licorice, but which may cause pseudohyperaldosteronism as a side effect. In the present invention, the fraction obtained through selective extraction of glycosides of liquiritigenin such as liquiritin and the like and purification by column chromatography using a synthetic adsorbent does not contain not only saponins such as glycyrrhizin and the like but also flavonoids such as isoliquiritin and the like, and thus can be used more preferably.

The differentiation promoter, proliferation promoter, and production promoter of the present invention can be prepared by adding as necessary general additives used in the field of preparation making to one or more kinds of glycosides of liquiritigenin, and according to a formulating means well known in the field of preparation making, for example, the method described in the Japanese Pharmacopoeia 17th edition, General Rules for Preparations [3] each preparation section and the like, and can be prepared as a liquid form such as solution, suspension, emulsion and the like; a semi-solid form such as gel, paste, cream and the like; a solid form such as powder, granule, tablet, capsule and the like, or the like.

Examples of the above-mentioned additive include excipient, binder, disintegrant, lubricant, coating agent, base, solvent, emulsifier, dispersing agent, suspending agent, stabilizer, thickening agent, pH adjuster, antioxidant, antiseptic, preservative, corrigent, sweetening agent, flavor, colorant and the like.

Examples of the excipient include silicic anhydride, calcium silicate, magnesium aluminosilicate, starch (cornstarch, potato starch, wheat starch etc.), saccharides (glucose, lactose, sucrose etc.), sugar alcohol (sorbitol, maltitol, mannitol etc.) and the like.

Examples of the binder include gelatin, sodium caseinate, starch and processed starch (cornstarch, hydroxypropyl starch, pregelatinized starch, partly pregelatinized starch etc.), cellulose and a derivative thereof (crystalline cellulose, hydroxypropylcellulose, carboxymethylcellulose etc.) and the like.

Examples of the disintegrant include povidone, crospovidone, cellulose and a derivative thereof (crystalline cellulose, methylcellulose etc.) and the like.

Examples of the lubricant include talc, synthetic aluminum silicate, magnesium silicate, calcium stearate, magnesium stearate and the like.

Examples of the coating agent include methacrylic acid copolymer (methacrylic acid-ethyl acrylate copolymer etc.), methacrylate copolymer (ethyl acrylate-methyl methacrylate copolymer, ethyl acrylate-methyl methacrylate-methacrylic acid trimethylammonium chloride ethyl copolymer etc.) and the like.

Examples of the base include hydrocarbon (liquid paraffin etc.), polyethylene glycol (macrogol 400, macrogol 1500 etc.) and the like.

Examples of the solvent include purified water, monovalent alcohol (ethanol etc.), polyhydric alcohol (propylene glycol, glycerol etc.) and the like.

Examples of the emulsifier include non-ionic surfactant (sorbitan fatty acid ester, glycerol fatty acid ester, polyoxyethylene sorbitan fatty acid ester, sucrose fatty acid ester etc.), anionic surfactant (sodium alkylsulfate, N-acylglutamate etc.), purified soybean lecithin and the like.

Examples of the dispersing agent include gum arabic, propyleneglycol alginate, non-ionic surfactant (polyoxyethylene hydrogenated castor oil, polyoxyethylene sorbitan fatty acid ester, polyoxyethylene polyoxypropylene glycol etc.), anionic surfactant (sodium alkylsulfate etc.) and the like.

Examples of the suspending agent include sodium alginate, non-ionic surfactant (polyoxyethylenelauryl ether, glycerol fatty acid ester, polyoxyethylene sorbitan fatty acid ester, propylene glycol fatty acid ester etc.) and the like.

Examples of the stabilizer include adipic acid, ethylenediamine tetraacetate (calcium disodium ethylenediamine tetraacetate, disodium ethylenediamine tetraacetate etc.), α-cyclodextrin, β-cyclodextrin and the like.

Examples of the thickening agent include water-soluble polymer (sodium polyacrylate, carboxyvinyl polymer etc.), polysaccharides (sodium alginate, xanthan gum, tragacanth etc.) and the like.

Examples of the pH adjuster include hydrochloric acid, phosphoric acid, acetic acid, citric acid, lactic acid, sodium hydroxide, sodium dihydrogenphosphate and the like.

Examples of the antioxidant include dibutylhydroxytoluene (BHT), butylhydroxyanisole (BHA), dl-α-tocopherol, erythorbic acid, propyl gallate and the like.

Examples of the preservative or the antiseptic include p-hydroxybenzoic acid ester (methyl p-hydroxybenzoate, etc.), benzoic acid, sodium benzoate, sorbic acid, potassium sorbate, lactic acid, sodium lactate and the like.

Examples of the corrigent include ascorbic acid, erythritol, disodium 5'-guanylate, citric acid, sodium L-glutamate, tartaric acid, DL-malic acid and the like.

Examples of the sweetening agent include aspartame, stevia, saccharin and the like.

Examples of the flavor include orange essence, l-menthol, d-borneol, vanillin, linalool and the like.

Examples of the colorant include tar pigment (Food Color Red No. 2, Food Color Blue No. 1, Food Color yellow No. 4 etc.), inorganic pigment (red iron oxide, yellow ferric oxide, titanium oxide etc.), natural dye (annatto dye, turmeric dye, carotenoid etc.) and the like.

One or more kinds of the above-mentioned additives can be used as necessary.

The differentiation promoter of the present invention can favorably promote the differentiation of chondrogenic progenitor cells into chondrocytes and promote the formation of chondrocytes.

In addition, the proliferation promoter of the present invention can promote the proliferation of differentiated chondrocytes.

Furthermore, the production promoter of the present invention can favorably promote the production of cartilaginous matrix by chondrocytes.

Therefore, the differentiation promoter, proliferation promoter, and production promoter of the present invention have an effective cartilage regeneration action and are effective in compensating for cartilage reduction or loss in tissues where cartilage is damaged or inflamed.

Also, the differentiation promoter and proliferation promoter of the present invention can be preferably used for culturing chondrocytes to compensate for tissues where cartilage is reduced or lost.

Therefore, the differentiation promoter, proliferation promoter, and production promoter of the present invention can be preferably used for the prophylaxis or treatment of symptoms and diseases caused by damage or inflammation of cartilage, or symptoms and diseases showing the loss of cartilage, such as osteoarthritis.

Furthermore, a glycoside of liquiritigenin contained in the differentiation promoter, proliferation promoter, and production promoter of the present invention is a component contained in licorice, which has been used as a Chinese herbal medicine since ancient times, and is a highly safe component.

Therefore, the differentiation promoter, proliferation promoter, and production promoter of the present invention can be continuously ingested or administered for a long period of time.

The content of glycoside of liquiritigenin in the differentiation promoter, proliferation promoter, and production promoter of the present invention is generally 0.00005 wt% to 100 wt%, preferably 0.0001 wt% to 100 wt%, more preferably 0.0002 wt% to 100 wt%, as the content of liquiritin.

The daily ingestion amount or dose of the differentiation promoter, proliferation promoter, and production promoter of the present invention is appropriately determined according to the sex, age of the target who receives application (hereinafter to be also referred to as "application target" in the present specification), the state and level of damage or inflammation of cartilage, or the state and level of loss of cartilage observed in the application target, and the form, administration method and the like of the differentiation promoter, proliferation promoter, and production promoter of the present invention. When the application target is a human adult, the amount is generally 2 mg to 4000 mg, preferably 20 mg to 2000 mg, more preferably 50 mg to 500 mg as the amount of liquiritin.

The above-mentioned amount may be ingested or administered once, or may be ingested or administered several times (e.g., 2 - 3 times) by portions per day.

The ingestion or dosing period of the differentiation promoter, proliferation promoter, and production promoter of the present invention is also appropriately determined according to the state and level of damage or inflammation of cartilage, or the state and level of loss of cartilage in the application target, and the like. Considering that glycoside of liquiritigenin is a component contained in licorice which has been used as a Chinese herbal medicine and the like since ancient times, and is highly safe, symptoms and diseases caused by damage or inflammation of cartilage, or symptoms and diseases showing the loss of cartilage, such as osteoarthritis, are symptoms and diseases caused by aging, dynamic load over a long period of time, and the like, or autoimmune diseases such as rheumatoid arthritis, and the like, it is preferable to continue ingestion or administration of the differentiation promoter, proliferation promoter, and production promoter of the present invention for a long period of time to prevent or improve such symptoms and diseases.

The differentiation promoter, proliferation promoter, and production promoter of the present invention can be formulated as a unit package form. In the present specification, the "unit package form" means a form of one or more units with a particular amount (e.g., ingestion amount or dose per one time etc.) as one unit is/are filled in one container or packed in a package. For example, a unit package form with ingestion amount or dose per one time as one unit is referred to as "unit package form for ingestion amount or dose per one time". A container or package used for the unit package form can be appropriately selected according to the form and the like of the differentiation promoter, proliferation promoter, and production promoter of the present invention. For example, paper container or bag, plastic container or bag, pouch, aluminum can, steel can, glass bottle, pet bottle, PTP (press through pack) package sheet and the like can be mentioned.

The application subject of the differentiation promoter, proliferation promoter, and production promoter of the present invention includes, for example, mammals (e.g., human, monkey, mouse, rat, guinea pig, hamster, rabbit, cat, dog, bovine, horse, donkey, swine, sheep, etc.), birds (e.g., duck, chicken, goose, turkey, etc.) and the like.

When the differentiation promoter, proliferation promoter, or production promoter of the present invention is applied to an animal other than human among the above-mentioned application target animals (hereinafter to be also simply referred to as "target animal"), the ingestion amount or dose of the differentiation promoter, proliferation promoter, or production promoter of the present invention can be appropriately set according to the kind, sex, body weight and the like of the target animal.

The differentiation promoter, proliferation promoter, and production promoter of the present invention has, as described above, a promoting action on the good differentiation of chondrogenic progenitor cells into chondrocytes, a promoting action on the proliferation of differentiated chondrocytes, and a promoting action on the production of cartilaginous matrix by chondrocytes, and functions as a cartilage regenerant.

Therefore, the present invention provides, as another embodiment, a regenerant of cartilage (hereinafter also to be referred to as "the regenerant of the present invention" in the present specification).

The regenerant of the present invention contains a glycoside of liquiritigenin as an active ingredient.

The glycoside of liquiritigenin contained in the regenerant of the present invention, the content, the dose or ingestion amount and the number of doses or ingestion per day, and the duration of administration or ingestion, of the regenerant of the present invention are as described above with respect to the differentiation promoter, proliferation promoter, and production promoter of the present invention.

The form, package form, and application target of the regenerant of the present invention are as described above with respect to the differentiation promoter, proliferation promoter, and production promoter of the present invention.

The differentiation promoter, proliferation promoter, or production promoter of the present invention can be provided as a pharmaceutical product (hereinafter to be also referred to as "the pharmaceutical product of the present invention" in the present specification) directly or after further adding above-mentioned general additives in the field of the preparation.

The pharmaceutical product of the present invention can have a dosage form of oral preparation such as tablet, coating tablet, chewable tablet, pill, capsule, microcapsule, granule, fine granule, powder, elixir, lemonade, syrup, suspension, emulsion, oral jelly and the like; injectable preparation such as injection such as solution, suspension, emulsion and the like, solid injection to be used by dissolving or suspending when in use, transfusion, sustainable injection and the like, and the like.

As mentioned above, glycoside of liquiritigenin to be contained in the differentiation promoter, proliferation promoter, and production promoter of the present invention is mainly obtained from licorice which has been utilized as Chinese herbal medicine since ancient times and is suitable for oral ingestion. Therefore, the pharmaceutical product of the present invention can be preferably provided as an oral preparation.

The pharmaceutical product of the present invention contains the differentiation promoter, proliferation promoter, or production promoter of the present invention in generally 0.001 wt% to 100 wt%, preferably 0.01 wt% to 100 wt%, more preferably 0.1 wt% to 100 wt%, as the content of liquiritin.

The pharmaceutical product of the present invention may further contain, as long as the characteristics of the present invention are not impaired, drugs used for mitigation of the symptoms of diseases caused by damage or inflammation of cartilage, or diseases showing the loss of cartilage, such as osteoarthritis, for example, analgesics such as acetaminophen and the like; non-steroidal anti-inflammatory agents such as loxoprofen, diclofenac, and the like.

The pharmaceutical product of the present invention can be preferably administered to those having symptoms and diseases caused by damage or inflammation of cartilage, or symptoms and diseases showing the loss of cartilage, such as osteoarthritis, and those having a high risk of developing symptoms and diseases caused by damage or inflammation of cartilage, or symptoms and diseases showing the loss of cartilage, due to aging, dynamic load over a long period of time, and the like.

The pharmaceutical product of the present invention can be preferably administered to the above-mentioned application target such that the ingestion amount or dose per day as converted to liquiritin amount is the above-mentioned ingestion amount or dose per day.

Furthermore, the differentiation promoter, proliferation promoter and production promoter of the present invention can be ingested by adding to various foods. The food to which the differentiation promoter, proliferation promoter and production promoter of the present invention is added is not particularly limited, and may be any as long as it is a food in the form generally served for meals or dessert.

For example, the differentiation promoter, proliferation promoter and production promoter of the present invention is added to drinks such as tea, beverage water and the like, and a suitable flavor is added when desired, whereby a functional drink can be provided.

More specifically, the differentiation promoter, proliferation promoter and production promoter of the present invention can be added, for example, to beverage water such as tea drinks, fruit drinks and the like; tea such as green tea, black tea, Chinese tea and the like; dairy products such as milk, yogurt and the like; confectionery such as jelly, biscuit, candy, caramel, and the like, and the like.

The differentiation promoter, proliferation promoter, and production promoter of the present invention is preferably added to the above-mentioned various foods in amounts to be ingested per day, such that the amount converted to the liquiritin amount is the above-mentioned ingestion amount per day.

The differentiation promoter, proliferation promoter, and production promoter of the present invention can be provided as a food (hereinafter to be also referred to as "the food of the present invention" in the present specification) directly or by adding general food additives as necessary and according to a general food production technique.

The food of the present invention can be prepared as various forms such as liquid (e.g., solution, suspension, emulsified liquid, etc.); semi-solid form (e.g., gel, cream, paste, etc.); solid form (e.g., powder, granule, sheet, capsule, tablet, etc.) and the like.

Furthermore, by adding the differentiation promoter, proliferation promoter, and production promoter of the present invention to various food starting materials and adding general food additives as necessary, various food forms such as beverage water (fruit juice drinks, sport drinks, coffee drinks, tea drinks etc.), tea (green tea, white tea, yellow tea, blue tea, black tea, dark tea, flower tea etc.), dairy product (lactic fermenting beverage, fermented milk, butter, cheese, yogurt, processed milk, defatted milk etc.), processed meat product (ham, sausage, hamburg etc.), fish pastry product (fish cake, tube-shaped fish sausage etc.), egg processed product (rolled omelet, egg tofu, Chawan-mushi etc.), soybean processed product (soybean paste, tofu, fermented soybean etc.), wheat processed product (bread, Japanese wheat noodles, pasta etc.), dried seafood (bonito, dried broth, dried young sardines, etc.), confectionery (cookie, biscuit, jelly, chewing gum, candy, caramel, ice cream, etc.), soup (miso soup, consommé soup, corn soup, potage, etc.), seasoning (dressing, Worcestershire sauce, mayonnaise etc.) and the like can be provided, and it is also possible to adapt a package form such as a bottled food, canned food or retort pouch food.

As the above-mentioned food additive, manufacturing agent (brine, binding agent etc.), thickening stabilizer (xanthan gum, sodium carboxymethylcellulose etc.), gelling agent (gelatin, agar, carrageenan etc.), gum base (vinyl acetate resin, jelutong, chicle etc.), emulsifier (glycerol fatty acid ester, sucrose fatty acid ester, saponin, lecithin etc.), preservative (benzoic acid, sodium benzoate, sorbic acid, potassium sorbate, ε-polylysine etc.), antioxidant (ascorbic acid, erythorbic acid, catechin etc.), glazing agent (shellac, paraffin wax, beeswax etc.), fungicide (thiabendazole, fludioxonil etc.), leavening agent (sodium hydrogen carbonate, glucono δ-lactone, alum etc.), sweetener (aspartame, acesulfame potassium, stevia etc.), bittering agent (caffeine, naringin, worm wood extract etc.), acidulant (citric acid, tartaric acid, lactic acid etc.), seasoning (sodium L-glutamate, disodium 5'-inosinate etc.), colorant (annatto dye, turmeric dye, gardenia dye etc.), flavor (synthetic flavor such as ethyl acetoacetate, anisealdehyde and the like, natural flavor such as orange, lavender and the like) and the like can be mentioned.

In the present invention, one or more kinds of the above-mentioned food additives can be used.

The food of the present invention contains the differentiation promoter, proliferation promoter, or production promoter of the present invention in generally 0.00005 wt% to 100 wt%, preferably 0.0001 wt% to 100 wt%, more preferably 0.0002 wt% to 99.9 wt%, as the content of liquiritin.

Furthermore, the food of the present invention may contain nutrients effective for damage to or decrease in cartilage, such as calcium, protein, chondroitin, glucosamine, and the like, and a food material containing such nutrients, as long as the characteristics of the present invention are not impaired.

The food of the present invention can be preferably ingested by those having symptoms and diseases caused by damage or inflammation of cartilage, or symptoms and diseases showing the loss of cartilage, such as osteoarthritis, those having a high risk of developing symptoms and diseases caused by damage or inflammation of cartilage, or symptoms and diseases showing the loss of cartilage, due to aging, dynamic load over a long period of time, and the like. In addition, it can be widely ingested by middle and old aged people, athletes, and the like for the purpose of preventing symptoms and diseases caused by damage or inflammation of cartilage, or symptoms and diseases showing the loss of cartilage.

Therefore, the food of the present invention can also be provided as food with health claims such as food for specified health uses, food with nutrient function claims, indicated functional food and the like, special purpose foods such as food for sick people, food for elderly people and the like, health supplement, dietary supplement and the like for preventing or improving symptoms and diseases caused by damage or inflammation of cartilage, or symptoms and diseases showing the loss of cartilage, such as osteoarthritis and the like.

The food of the present invention is preferably ingested by the above-mentioned application target such that the ingestion amount per day as converted to the liquiritin amount is the above-mentioned ingestion amount per day.

The present invention can also provide a commercial package containing the above-mentioned differentiation promoter, proliferation promoter and production promoter of the present invention and a written matter associated therewith, the written matter stating that the differentiation promoter, proliferation promoter and production promoter can or should be used for promoting differentiation into chondrocyte, promoting proliferation of chondrocytes, promoting production of cartilaginous matrix or regeneration of cartilage, or prophylaxis or improvement of a symptom or disease caused by damage or inflammation of cartilage, or a symptom or disease showing the loss of cartilage.

Furthermore, the present invention provides a method for promoting differentiation into chondrocytes or proliferation of chondrocytes, or production of cartilaginous matrix in a target animal in need of the promotion of differentiation into chondrocytes or proliferation of chondrocytes, or production of cartilaginous matrix (hereinafter also to be referred to as "the promotion method of the present invention" in the present specification).

The promotion method of the present invention includes ingestion by or administration to, a target animal in need of promotion of differentiation into chondrocyte, proliferation of chondrocyte, or production of cartilaginous matrix, of glycoside of liquiritigenin in an amount effective for promoting differentiation into chondrocyte, proliferation of chondrocyte, or production of cartilaginous matrix in the target animal.

The promotion method of the present invention can be preferably used for culturing chondrocytes to compensate for tissues where cartilage is reduced or lost, and regenerating cartilage where cartilage is reduced or lost.

Therefore, the present invention provides a method for regenerating cartilage in a target animal in need of regenerating cartilage (hereinafter also to be referred to as "the regeneration method of the present invention" in the present specification).

The regeneration method of the present invention includes ingestion by or administration to, a target animal in need of regeneration of cartilage, of glycoside of liquiritigenin in an amount effective for regenerating cartilage in the target animal.

Furthermore, the present invention also provides a method for preventing or improving cartilage reduction or loss in a target animal in need of preventing or improving cartilage reduction or loss, and a method for preventing or improving a symptom or disease caused by damage or inflammation of cartilage, or a symptom or disease showing the loss of cartilage in a target animal in need of preventing or improving a symptom or disease caused by damage or inflammation of cartilage, or a symptom or disease showing the loss of cartilage (hereinafter also to be referred to as "the preventing or improving method of the present invention" in the present specification).

The preventing or improving method of the present invention includes ingestion by or administration to, a target animal in need of preventing or improving cartilage reduction or loss or a target animal in need of preventing or improving a symptom or disease caused by damage or inflammation of cartilage, or a symptom or disease showing the loss of cartilage, of glycoside of liquiritigenin in an amount effective for preventing or improving cartilage reduction or loss in the target animal or in an amount effective for preventing or improving a symptom or disease caused by damage or inflammation of cartilage, or a symptom or disease showing the loss of cartilage in the target animal.

The preventing or improving method of the present invention can be preferably used for the prophylaxis or treatment of symptoms and diseases caused by damage or inflammation of cartilage, or symptoms and diseases showing the loss of cartilage, such as osteoarthritis and the like.

The target animal in the above-mentioned promoting method, the regeneration method and the preventing or improving method of the present invention includes mammal (e.g., human, monkey, mouse, rat, guinea pig, hamster, rabbit, cat, dog, bovine, horse, donkey, swine, sheep etc.), birds (e.g., duck, chicken, goose, turkey etc.) and the like.

In the case of human, the promotion method of the present invention, the regeneration method of the present invention, and the preventing or improving method of the present invention can be preferably applied to patients having a symptom or disease caused by damage or inflammation of cartilage, or a symptom or disease showing the loss of cartilage, or those having a high risk of developing symptoms and diseases caused by damage or inflammation of cartilage, or symptoms and diseases showing the loss of cartilage, due to aging, dynamic load over a long period of time, and the like.

The effective amount of glycoside of liquiritigenin in the promotion method of the present invention, the regeneration method of the present invention, and the preventing or improving method of the present invention is determined according to the kind, age, gender, the state and level of inflammation, damage or loss of cartilage, and the like of the target animal. An amount similar to the above-mentioned ingestion amount or dose of the differentiation promoter, proliferation promoter, and production promoter of the present invention for a human or a target animal other than human can be ingested or administered at the frequency and period mentioned above.

The ingestion or administration method of glycoside of liquiritigenin in the promotion method, the regeneration method and the preventing or improving method of the present invention includes oral ingestion or oral administration, injection, administration by infusion and the like. Oral ingestion or oral administration is preferable since convenient ingestion or administration is possible without the need to perform under the guidance and supervision of a doctor at a medical institution.

### [Example]

The present invention is explained in more detail by in the following by Experimental Example and the like.

In the following Experimental Examples, a pure product manufactured by FUJIFILM Wako Pure Chemical Corporation was used as liquiritin,.

### [Experimental Example 1] Examination of effects of liquiritin on chondrocyte differentiation, chondrocyte proliferation, and cartilage matrix production

Using chondrogenic progenitor ATDC5 cells, the effects of liquiritin on the differentiation of chondrogenic progenitor cells into chondrocytes, proliferation of chondrocytes, and production of cartilaginous matrix were investigated as follows.

### (1) Cultivation of chondrogenic progenitor cells and induction of differentiation into chondrocytes

ATDC5 cells (obtained from RIKEN BioResource Research Center) were seeded at 1×10⁴ cells/well in a microplate containing 0.5 mL of Dulbecco's modified Eagle medium (DMEM)/Ham's F-12 medium (manufactured by FUJIFILM Wako Pure Chemical Corporation), and cultured at 37°C in the presence of 5% by volume carbon dioxide.

On days 3, 6, 9, 12, 15, 18 and 21 of culture, transferrin and sodium selenite, which are differentiationinducing reagents, were each added to the aforementioned medium at final concentrations of 10 µg/mL and 30 nM, respectively, and further, the medium was replaced with a medium to which liquiritin was added at each final concentration of 5 pM, 10 pM, or 20 pM, and the culture was continued.

The case of same treatment without adding liquiritin (liquiritin concentration = 0 µM) was used as a control.

### (2) Detection of glycosaminoglycan by Alcian blue staining

On day 24 of culture, the cells were fixed with 95% by volume ethanol, and then stained with Alcian blue staining solution (prepared with 1% Alcian blue and 3% acetic acid aqueous solution, pH=2.5), and glycosaminoglycan was detected.

Then, the Alcian blue dye of the stained cultured cells was solubilized with 6M guanidine hydrochloride, and the absorbance at 620 nm was measured using a plate reader (Infinite 200 PRO, manufactured by Tecan Japan Co., Ltd., for each liquiritin concentration and control, n=4). The measurement results are shown in Fig. 1 as mean±standard deviation based on relative values when the absorbance in the control is 100. As regards the measurement results, an unpaired t-test was performed between each group added with liquiritin at the above-mentioned concentration and the control.

As shown in Fig. 1, it was found that addition of not less than 10 µM of liquiritin increased glycosaminoglycan stained with Alcian blue in cultured cells, and addition of 20 µM of liquiritin resulted in a significant increase in the absorbance compared to the control (P<0.05).

### (3) Quantification of intracellular sulfated glycosaminoglycan

On day 24 of culture, the cells were washed with 10 mM phosphate buffer (pH=7.4), 0.2 mL of 100 ug/mL proteinase K solution (prepared with an aqueous solution (pH=7.1) containing 50 mM sodium phosphate and 5 mM ethylenediaminetetraacetic acid (EDTA)) was added, and the cells were incubated overnight at 37°C to digest the cells. The cell digestion fluid was centrifuged, and the obtained supernatant was used as a sample.

Each sample (20 µL) was blended with 200 µL of a 16 mg/L dimethylmethylene blue solution (prepared with an aqueous solution (pH=3.0) containing 40 mM glycine, 27 mM sodium chloride, and 10 mM acetic acid), and the absorbance at 525 nm was measured and sulfated glycosaminoglycan in the sample was quantified (n=4 for each). The quantification results are shown in Fig. 2 as mean±standard deviation. As regards the quantification results, an unpaired t-test was performed between each group added with liquiritin at the above-mentioned concentration and the control.

As shown in Fig. 2, the amount of sulfated glycosaminoglycan contained in cell digestive fluid increased according to the concentration of liquiritin added, and a significant increase was observed with 5 µM to 20 µM of liquiritin compared to the control (P<0.01).

### (4) Measurement of number of chondrocytes

The content of DNA in the above-mentioned sample was measured using a DNA quantification kit ("DNA Assay Kit", manufactured by Cosmo Bio Company, Limited) (n=4 for each) and used as an index of cell number. The measurement results are shown in Fig. 3 as mean±standard deviation. As regards the measurement results, an unpaired t-test was performed between the group added with liquiritin and the control.

In addition, on day 24 of culture, the cells were washed with 10 mM phosphate buffered saline (pH=7.4), the cells were detached using 0.25(w/v)% trypsin-1 mM tetrasodium ethylenediaminetetraacetate solution (manufactured by FUJIFILM Wako Pure Chemical Corporation), the number of cells in the cell suspension was observed under an inverted culture microscope (CK40, manufactured by Olympus Corporation) (magnification=10x), and the number of cells was measured (n=4 for each). The measurement results are shown in Fig. 4 as mean±standard deviation. As regards the measurement results, an unpaired t-test was performed between each group added with liquiritin at the above-mentioned concentration and the control.

As shown in Fig. 3, the content of DNA in the cell digestive fluid, which was used as an index of cell number, increased with the addition concentration of liquiditin (at 10 µM and 20 µM, significant at P<0.01 compared to control).

As shown in Fig. 4, the cell number on day 24 of culture also increased with the addition concentration of liquiritin (at 10 µM and 20 µM, significant at P<0.05 compared to control).

The above results suggest that liquiritin can promote the differentiation of chondrogenic progenitor cells into chondrocytes at concentrations of 10 µM to 20 µM, and can promote the production of glycosaminoglycan by chondrocytes at concentrations as low as 5 µM. In addition, it was suggested that liquiritin promotes the proliferation of chondrocytes at a concentration of 10 µM to 20 µM and does not exhibit cytotoxicity within the aforementioned concentration range.

Therefore, it was suggested that liquiritin promotes differentiation of chondrogenic progenitor cells into chondrocytes at very low concentrations, further promotes the proliferation of chondrocytes and production of cartilaginous matrix, and can improve the cartilage regeneration action.

### [Industrial Applicability]

As described above, the present invention can provide promoters for differentiation into chondrocytes, promoters for proliferation of chondrocytes, and promoters for cartilaginous matrix production, that are superior in a promoting action on differentiation into chondrocytes, a promoting action on proliferation of chondrocytes, and a promoting action on cartilaginous matrix production.

The promoters for differentiation into chondrocytes, promoters for proliferation of chondrocytes, and promoters for cartilaginous matrix production of the present invention have high stability and high safety and are effective for regeneration of cartilage, and useful for the prophylaxis or improvement of symptoms and diseases caused by damage or inflammation of cartilage, or symptoms and diseases showing the loss of cartilage such as osteoarthritis and the like.

This application is based on a patent application No. 2021-152615 filed in Japan, the contents of which are incorporated in full herein.

## Claims

1. A promoter for differentiation into chondrocytes, comprising a glycoside of liquiritigenin.

2. The promoter according to claim 1, wherein the glycoside of liquiritigenin is liquiritin.

3. A promoter for chondrocyte proliferation, comprising a glycoside of liquiritigenin.

4. The promoter according to claim 3, wherein the glycoside of liquiritigenin is liquiritin.

5. A cartilaginous matrix production promoter comprising a glycoside of liquiritigenin.

6. The promoter according to claim 5, wherein the glycoside of liquiritigenin is liquiritin.

7. A cartilage regenerant comprising a glycoside of liquiritigenin.

8. The regenerant according to claim 7, wherein the glycoside of liquiritigenin is liquiritin.

9. A pharmaceutical product for the prophylaxis or improvement of a symptom or disease caused by damage or inflammation of cartilage, or a symptom or disease showing the loss of cartilage, comprising the differentiation promoter according to claim 1 or 2.

10. The pharmaceutical product according to claim 9, wherein the symptom or disease caused by damage or inflammation of cartilage, or the symptom or disease showing the loss of cartilage is osteoarthritis.

11. A pharmaceutical product for the prophylaxis or improvement of a symptom or disease caused by damage or inflammation of cartilage, or a symptom or disease showing the loss of cartilage, comprising the proliferation promoter according to claim 3 or 4.

12. The pharmaceutical product according to claim 11, wherein the symptom or disease caused by damage or inflammation of cartilage, or the symptom or disease showing the loss of cartilage is osteoarthritis.

13. A pharmaceutical product for the prophylaxis or improvement of a symptom or disease caused by damage or inflammation of cartilage, or a symptom or disease showing the loss of cartilage, comprising the production promoter according to claim 5 or 6.

14. The pharmaceutical product according to claim 13, wherein the symptom or disease caused by damage or inflammation of cartilage, or the symptom or disease showing the loss of cartilage is osteoarthritis.

15. A food for the prophylaxis or improvement of a symptom or disease caused by damage or inflammation of cartilage, or a symptom or disease showing the loss of cartilage, comprising the differentiation promoter according to claim 1 or 2.

16. The food according to claim 15, wherein the symptom or disease caused by damage or inflammation of cartilage, or the symptom or disease showing the loss of cartilage is osteoarthritis.

17. A food for the prophylaxis or improvement of a symptom or disease caused by damage or inflammation of cartilage, or a symptom or disease showing the loss of cartilage, comprising the proliferation promoter according to claim 3 or 4.

18. The food according to claim 17, wherein the symptom or disease caused by damage or inflammation of cartilage, or the symptom or disease showing the loss of cartilage is osteoarthritis.

19. A food for the prophylaxis or improvement of a symptom or disease caused by damage or inflammation of cartilage, or a symptom or disease showing the loss of cartilage, comprising the production promoter according to claim 5 or 6.

20. The food according to claim 19, wherein the symptom or disease caused by damage or inflammation of cartilage, or the symptom or disease showing the loss of cartilage is osteoarthritis.
